(19) Europäisches Patentamt
European Patent Office
Office européen des brevets

(11) Publication number : 0 490 615 A1

(12) # EUROPEAN PATENT APPLICATION

(21) Application number : 91311438.5

(51) Int. Cl.[5] : C07D 311/62

(22) Date of filing : 09.12.91

(30) Priority : 10.12.90 GB 9026792

(43) Date of publication of application :
17.06.92 Bulletin 92/25

(84) Designated Contracting States :
AT BE CH DE DK ES FR GB GR IT LI LU NL SE

(71) Applicant : IdB Holding S.p.A.
Via Ripamonti, 99
I-20141 Milano (IT)

(72) Inventor : Nizzola, Rita, IdB Holding SpA
via Ripamonti 99
I-20141 Milan (IT)
Inventor : Gabetta, Bruno, IdB Holding SpA
via Ripamonti 99
I-20141 Milan (IT)

(74) Representative : Ritter, Stephen David et al
Mathys & Squire 10 Fleet Street
London EC4Y 1AY (GB)

(54) **Process for synthesising anthocyanidins.**

(57) A process is provided for producing anthocyanidins of formula III

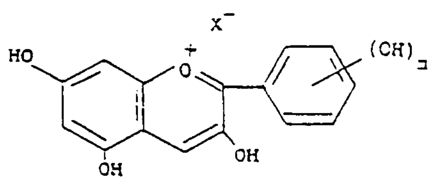

(III)

wherein m represents an integer from 1 to 3, and $X^-$ represents an anion, which involves reducing a protected flavonol of formula

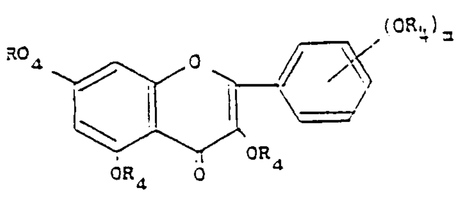

(IV)

where each $R^4$ represents a $C_1$#SB-#$_{10}$
hydrocarbyl group and m is 1 to 3 and converting $OR^4$ groups to hydroxy groups.



Jouve, 18, rue Saint-Denis, 75001 PARIS

This invention relates to a process for synthesising anthocyanidins.

Anthocyanidins are polyphenolic compounds which occur widely in nature in the form of glycosides (anthocyanins) and which are responsible for the typical colouring of numerous flowers and fruits. Some anthocyanidins are endowed with cicatrising, anti-inflammatory, vasoprotective, hypolipaemic, hypocholesterolaemic and hypoglycaemic activity (see e.g. U K Patent Specification No. 1,589,294 in the name of Inverni della Beffa). Examples of anthocyanidins include cyanidin (Ia), pelargonidin (Ib) and delphinidin (Ic).

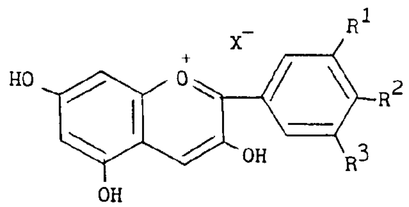

(Ia, Ib, Ic)

wherein

Ia $R^1=R^2=OH$, $R^3=H$ (cyanidin)
Ib $R^1=R^3=H$, $R^2=OH$ (pelargonidin)
Ic $R^1=R^2=R^3=OH$ (delphinidin)

and $X^-$ represents an anion, e.g. $Cl^-$.

Anthocyanidins can be prepared by hydrolysis of anthocyanin-containing extracts and purification of the resulting mixtures of anthocyanidins by chromatographic techniques. Alternatively, they may be prepared by total synthesis, for example according to the procedure described in UK Patent Application No. 8814603.0 in the name of Inverni della Beffa. (EP-A- 0 348 121)

A third method consists of a semisynthesis starting from natural compounds (flavonols) containing the 2-phenyl-3-hydroxy-benzopyran-4-one nucleus. With regard to this latter approach, various procedures are known which involve the transformation of flavonols, for example compounds IIa, IIb or IIc

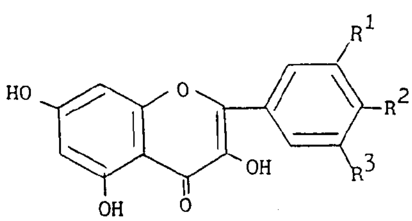

(IIa, IIb, IIc)

wherein

IIa $R^1=R^2=OH$, $R^3=H$ (quercetin)
IIb $R^1=R^3=H$, $R^2=OH$ (kaempferol)
IIc $R^1=R^2=R^3=OH$ (myricetin)

into anthocyanidins Ia, Ib, or Ic by the action, in the presence of mineral acids, of metals such as sodium amalgam or magnesium in the presence of mercury (T. A. Geissman, The Chemistry of Flavonoid Compounds, Pergamon Press, 1962, page 264).

A further semisynthesis involves the reductive acetylation of flavonols with zinc dust and sodium acetate in acetic anhydride. This procedure leads to a mixture of acetylated products from which anthocyanidins are formed by treatment with hydrochloric acid (H. Aft, R.R. Grant and R.J. Molyneux, Tetrahedron 23, 1963 (1967)).

None of the methods described in the literature can be used on an industrial scale because of the low yields and the difficulty of isolating the anthocyanidins from the reaction mixtures. They are essentially used for analytical purposes, in particular for the detection of the flavonol nucleus.

In a further semisynthetic process for the production of cyanidin (Ia), quercetin (IIa) esters are reduced using hydrides and the pyran ring is then aromatized in an acid medium (Japanese Kokai Tokkyo Koho 80 111,418).

This method allows cyanidin chloride to be obtained in good yields. However, nevertheless, the protection of the hydroxyl groups of quercetin by formation of esters has the disadvantage of requiring use of a great quantity of reducing agent, because the ester functions are also reduced by the hydride. In practice, 13 moles of reducing agent need to be employed per mole of quercetin.

The present invention provides a novel process of the semisynthetic type for the preparation of anthocyanidins (I) and in particular of the anthocyanidins Ia, Ib and Ic which avoids the aforementioned disadvantages.

According to the present invention there is provided a process for producing a compound of formula III

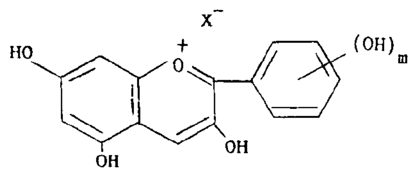

(III)

wherein m represents an integer from 1 to 3, and $X^-$ represents an anion, which comprises (i) reducing a protected flavonol of formula IV

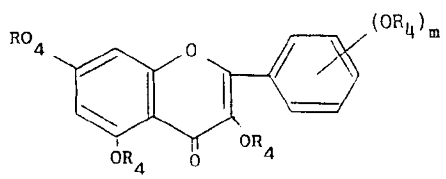

(IV)

wherein each $R^4$ (which may be the same or different) represents $C_{1-10}$ hydrocarbyl group, m represents an integer from 1 to 3 to form an intermediate of formula (V)

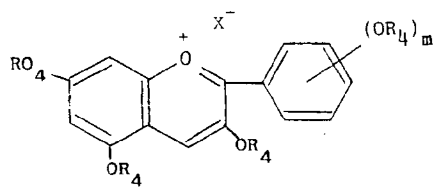

(V)

and (ii) converting the groups $OR^4$ to hydroxy groups

More specifically, there is provided a process for producing a compound of fomula Ia, Ib or Ic,

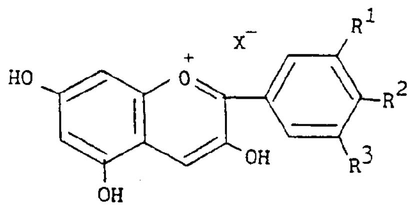

(Ia, Ib, Ic)

wherein

Ia $R^1$=$R^2$=OH, $R^3$=H (cyanidin)

Ib $R^1$=$R^3$=H, $R^2$=OH (pelargonidin)

Ic $R^1$=$R^2$=$R^3$=OH (delphinidin)

and $X^-$ represents an anion, e.g. $Cl^-$

which comprises (i) reacting a protected flavonol of formula IVa, IVb or IVc

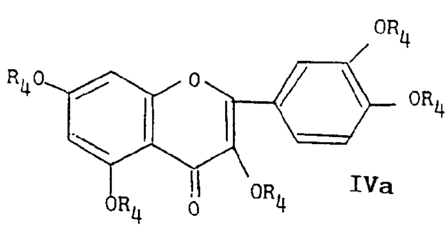

IVa

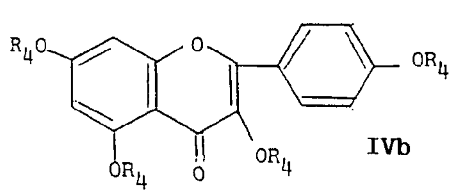

IVb

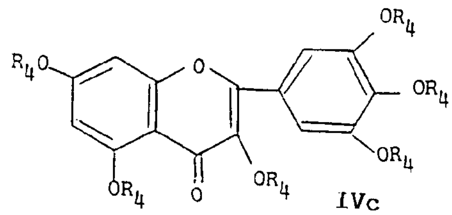

IVc

wherein each $R^4$, which may be the same or different represents $C_{1-10}$ hydrocarbyl to form an intermediate of formula Va, Vb or Vc

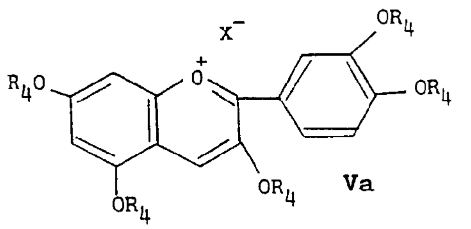

Va

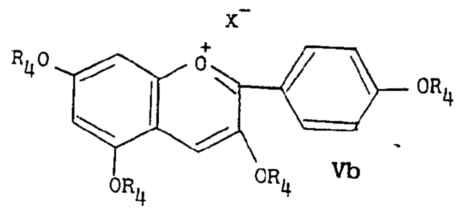

Vb

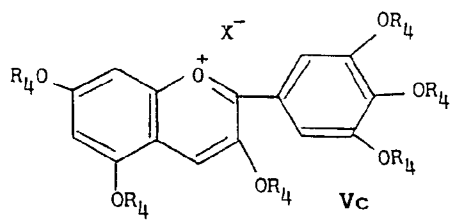

Vc

wherein each $R^4$ is as defined above and (ii) converting the groups $OR^4$ to hydroxy groups.

Examples of hydrocarbyl groups represented by $R^4$ include aliphatic groups, e.g. $C_{1-6}$ alkyl groups and aromatic groups, e.g. $C_{7-10}$ aralkyl groups.

The reduction step (i) is carried out using a metal hydride, particularly an aluminium hydride, as reducing agent. The reduction step is preferably carried out in the presence of an inert organic solvent.

More specifically, the process of the invention provides for protection of the hydroxyl functions of the flavonols (IV) as ethers. The flavonols, thus transformed into ether derivatives, are then reduced, preferably using an aluminium hydride, for example sodium dihydro-bis-(2-methoxyethanolate-O,O')-aluminate.

Reaction between a flavool and an aluminium hydride will usually result in formation of an aluminium adduct. On hydrolysis of this adduct, for example by treatment with aqueous acid, the pyran ring is aromatized to yield the intermediate of formula V.

In step (ii), the phenolic functions are restored by two methods. The first method (Method A) requires the use of a Lewis acid such as, for example, boron trichloride in an aprotic solvent (for example methylene chloride). An alternative method (Method B) consists in the use of aqueous hydrochloric acid in the presence of a phase transfer catalyst as activator. Both methods give high yields of anthocyanidins I.

The protection of the phenolic functions as ethers makes it possible greatly to reduce the quantity of hydride required for the reduction, inasmuch as the ether functions do not consume the reagent. The phenolic functions of the flavonols (IIa, IIb, IIc) can be protected as alkyl ethers by heating with an alkyl halide in a polar aprotic solvent, for example dimethylformamide, in the presence of potassium carbonate or another inorganic base. The halides used can be methyl, ethyl, n-propyl, i-propyl and benzyl chloride, bromide or iodide.

Isopropyl groups are the preferred protecting groups, since they can be quantitatively removed under relatively mild hydrolysis conditions.

The reduction reaction may be carried out at between 0° and 20°C by adding a solution of the protected flavonol (for example the preferred perisopropoxylated flavonol referred to above) in an aprotic solvent to the hydride dissolved in the same solvent. Examples of solvents which may used include toluene, tetrahydrofuran, dioxane and ethers.

The reaction mixture may then be diluted with acid, for example aqueous hydrochloric acid, and extracted

with a water-immiscible solvent, for example ethyl acetate. After evaporation of the organic phase, the removal of the ether groups can be carried out so as to regenerate the intermediates of formulae V, Va, Vb, Vc) with an acid.

For example, regeneration of the phenolic functions can be effected in an anhydrous medium by using a Lewis acid which is suitable for the hydrolysis of ether functions, for example boron trichloride (Method A). In this case, a solution of the reduced product in methylene chloride can be treated at a temperature of between -70°C and -20°C with boron trichloride dissolved in the same solvent. The reaction is completed by allowing the temperature to rise slowly up to 20°C. The excess reagent is hydrolysed by adding water. The crude anthocyanidin precipitates from the two-phase mixture in the form of the chloride which is purified by crystallization from methanol/concentrated aqueous hydrochloric acid.

The phenolic functions can also be liberated by heating in aqueous acids. The hydrolysis can be carried out in the presence of hydrochloric or hydrobromic acid in water, alcohols or acetic acid, but the removal of the protective groups is slow and incomplete. It has been found that (Method B), in the presence of phase transfer catalysts, such as, for example, hexadecylpyridinium chloride or hexadecyltrimethylammonium chloride, used in a quantity of 0.2 to 0.3 mole for each ether function present, the hydrolysis of the ether functions proceeds rapidly and with high yields.

By both of the Methods A and B, the anthocyanidins can be prepared from flavonols in yields and a quality comparable with those obtained by the use of the total synthesis processes referred to above.

The semisyntheses of cyanidin chloride (Ia), pelargonidin chloride (Ib) and delphinidin chloride (Ic) from the flavonols quercetin (IIa), kaempferol (IIb) and myricetin (IIc) respectively will now be described by way of example.

**Example 1 - Synthesis of cyanidin chloride (1a) (Method A)**

A solution of 50 g of quercetin (IIa) dihydrate in 350 ml of dimethylformamide is treated for 12 hours with stirring at 60°C with 275 g of anhydrous potassium carbonate and 165 ml of 2-bromopropane. After cooling to 20°C, the mixture is poured into ice water and extracted with ethyl acetate. The organic phase is dried and evaporated at reduced pressure, and the residue is crystallized from acetone. The crystallized solid (70 g) is dissolved in 800 ml of anhydrous toluene and added in the course of 30 minutes to 245 ml of a 20% solution of sodiumdihydro-bis-(2-methoxyethanolate-O,O')-aluminate in toluene precooled to 0-5°C under an inert atmosphere. The reaction mixture is left for 1 hour at 0°C and for 3 hours at 20°C and then poured into 3 l of ice water in the presence of 1 l of ethyl acetate. The mixture is acidified with concentrated hydrochloric acid to pH 1 and the organic phase is washed with water, dried and evaporated at reduced pressure. This gives 70 g of residue, which are dissolved in 850 ml of methylene chloride. 960 ml of a 1 M solution of boron trichloride in methylene chloride are added to the solution cooled to -30°C. The temperature is allowed to rise to 20°C and the mixture is then diluted with 1.5 l of cold water. The mixture is left to stand overnight, and the solid which has precipitated is filtered off and crystallized from methanol/concentrated hydrochloric acid. This gives 34.8 g of cyanidin chloride (Ia) in the form of the monohydrate (yield 69%) identical to a reference sample; melting point > 300°C (decomposition); E%cm 1215 at 540 nm (MeOH); purity by HPLC 98.7%.

**Example 2 - Synthesis of cyanidin chloride (Ia) (method B)**

The reduction product (70 g) obtained as described in Example 1 is treated with 3 l of concentrated hydrochloric acid containing 47g of cetylpyridinium chloride. The reaction mixture is heated under reflux with stirring until the ether functions have been completely hydrolysed. The mixture is cooled and the solid which has precipitated is filtered off and washed with acid water and ethyl acetate and recrystallized from methanol/concentrated hydrochloric acid. This gives 31 g of cyanidin chloride (Ia) monohydrate (yield 61%) identical to a reference sample; melting point > 300°C (decomposition); E%cm 1209 at 540 nm (MeOH); purity by HPLC 98.2%.

**Example 3 - Synthesis of pelargonidin chloride (Ib) - (Method A)**

A solution of 0.50 g of kaempferol (IIb) in 5 ml of dimethylformamide is treated for 12 hours with stirring at 60°C with 1.9 g of anhydrous potassium carbonate and 1.3 ml of 2-bromopropane. The mixture is cooled to 20°C, poured into water and extracted with ethyl acetate. The organic phase is dried and evaporated at reduced pressure: this gives 0.72 g of residue which is dissolved in 15 ml of toluene and the solution is added to 6 ml of a 24% solution of sodium dihydro-bis-(2-methoxyethanolate-O,O')-aluminate in toluene cooled to 0°C in an inert gas atmosphere.

The reaction mixture is left for 2 hours at 20°C with stirring. It is poured into ice water, acidified with concentrates hydrochloric acid to pH 1 and extracted with ethyl acetate. The organic phase is washed with water, dried and evaporate at a reduced pressure. This gives 0.70 g of residue which is dissolved in 30 ml of methylene chloride.

10 ml of a 1 M solution of boron trichloride in methylene chloride are added to the solution cooled to -40°C. The temperature is allowed to rise to 20°C, 40 ml of cold water are then added, and a solid separates out which is recovered by filtration and crystallized from methanol/concentrated hydrochloric acid. This gives 370 g of pelargonidin chloride (1b) in the form of the monohydrate (yield 65%) identical to a reference sample; melting point 302°C (decomposition); E%cm 1120 at 525 nm (MeOH); purity by HPLC 98.2%.

**Example 4 - Synthesis of delphinidin chloride (Ic) (Method A)**

3 g of anhydrous potassium carbonate and 2.5 ml of 2-bromopropane are added to a solution of 0.56 g of myricetin (2c) in 7 ml of dimethylformamide, and the reaction mixture is heated for 12 hours at 60°C. It is cooled to 20°C, poured into ice water and extracted with ethyl acetate, and the combined extraction phases are dried and evaporated at reduced pressure: this gives 0.88 g of residue which is dissolved in 40 ml of toluene and added to 6 ml of a 24% solution of sodium dihydro-bis-(2-methoxyethanolate-O,O′)-aluminate in toluene precooled to 0°C, in an inert gas atmosphere. The reaction mixture is left to stand for 2 hours at 20°C. It is poured into ice water, acidified with concentrated hydrochloric acid to pH 1 and extracted with ethyl acetate. The solvent is removed by evaporation from the dried extraction phase: this gives 0.85 g of residue which is dissolved in 40 ml of methylene chloride. 12 ml of a 1 M solution of boron trichloride in methylene chloride are added to the solution cooled to -40°C. The temperature is allowed to rise to 20°C and 50 ml of cold water are added: a solid separates out which is recovered by filtration and crystallized from methanol/concentrated hydrochloric acid. This gives 390 mg of delphinidin chloride (1c) monohydrate (yield 62%) identical to a reference sample; melting point 250°C; E%cm 1209 at 545 nm (MeOH); purity by HPLC 98.2%.

## Claims

1. A process for producing a compound of formula III

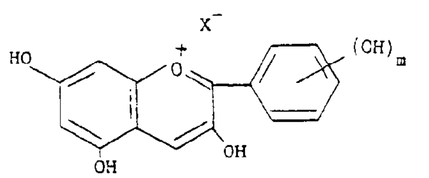

(III)

wherein n and m each represents an integer from 1 to 3, and $X^-$ represents an anion, which comprises (i) reducing a protected flavonol of formula IV

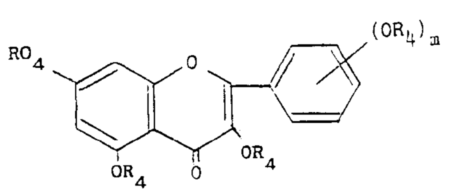

(IV)

wwherein each $R^4$ (which may be the same or different) represents $C_{1-10}$ hydrocarbyl group, and m represents an integer from 1 to 3, using a metal hydride, to form an intermediate of formula (V)

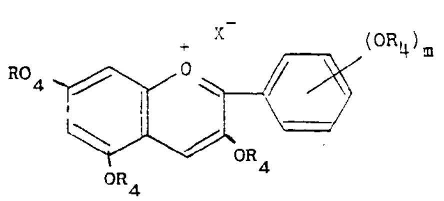

(V)

and (ii) converting the groups $OR^4$ to hydroxy groups.

**2.** A process according to Claim 1 for producing a compound of fomula Ia, Ib or Ic,

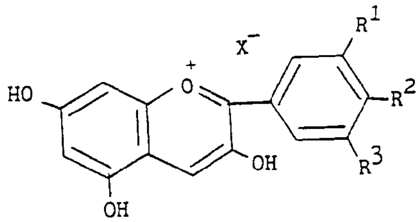

(Ia, Ib, Ic)

wherein

1a $R^1=R^2=OH$, $R^3=H$ (cyanidin)

1b $R^1=R^3=H$, $R^2=OH$ (pelargonidin)

1c $R^1=R^2=R^3=OH$, (delphinidin)

and $X^-$ represents an anion,

which comprises (i) reducing a protected flavonol of formula IVa, IVb or IVc

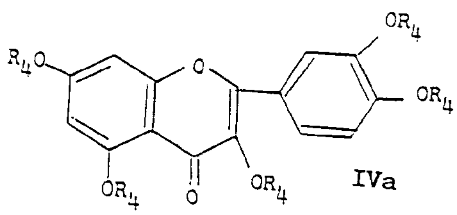

IVa

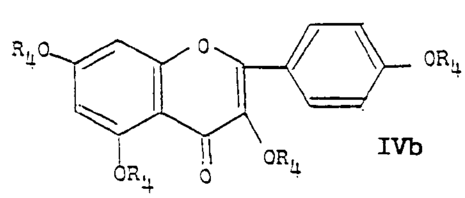

IVb

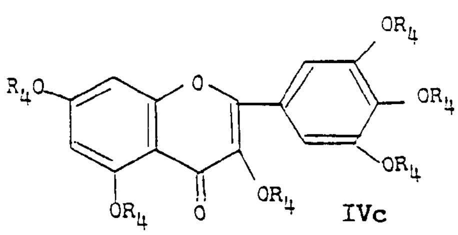

IVc

wherein each $R^4$ which may be the same or different represents $C_{1-10}$ hydrocarbyl to form an intermediate of formula Va, Vb or Vc

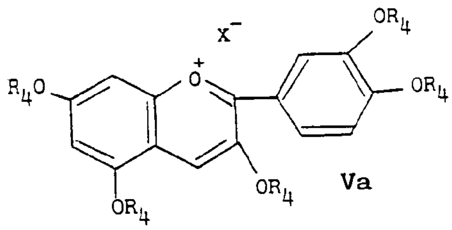

Va

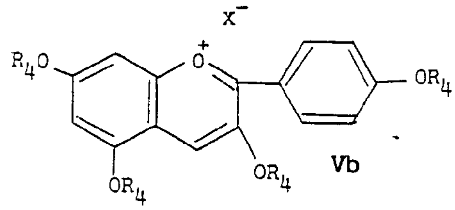

Vb

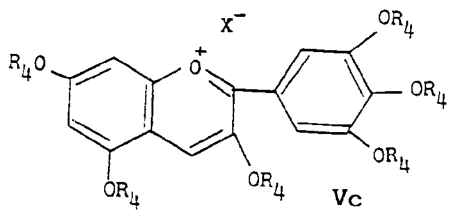

Vc

wherein each $R^4$ is as defined above and (ii) converting the groups $OR^4$ to hydroxy groups.

3. A process according to Claim 1 or Claim 2 wherein hydrocarbyl groups represented by $R^4$ are selected from $C_{1-6}$ alkyl groups and $C_{7-10}$ aralkyl groups.

4. A process according to Claim 3 wherein hydrocarbyl groups represented by $R^4$ are isopropyl groups.

5. A process according to any preceding claim wherein reduction step (i) is carried out in the presence of an inert organic solvent.

6. A process according to any preceding claim wherein reduction step (i) is carried out using an aluminium hydride as reducing agent in the presence of an inert organic solvent.

7. A process according to Claim 5 wherein reaction between the protected flavanol and the aluminium hydride results in formation of an aluminium adduct which is hydrolysed by treatment with aqueous acid to yield the intermediate of formula V, Va, Vb or Vc.

8. A process according to any preceding claim wherein in step (ii) the intermediate of formula V, Va, Vb, or Vc is contacted with a Lewis acid in an aprotic solvent.

9. A process according to any of Claims 1 to 6 wherein in step (ii) the intermediate of formula V, Va, Vb, or Vc is contacted with an aqueous acid in the presence of a phase transfer catalyst as activator.

European Patent Office

# EUROPEAN SEARCH REPORT

Application Number

EP 91 31 1438

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A<br><br><br><br>D | CHEMICAL ABSTRACTS, vol. 94, no. 10, 9 March 1981, Columbus, Ohio, US; abstract no. 71489T, page 380 ;<br>& JP-A-80 111 418 (SANTEN PHARMACEUTICAL) 28-08-1980<br>--- | 1,2,6 | C07D311/62 |
| A | GB-A-1 253 (E.R. WATSON et al.) (A.D. 1915)<br>* the whole document *<br>----- | 1,2 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 17 FEBRUARY 1992 | ENGLISH R.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)